(19) Europäisches Patentamt

European Patent Office

Office européen des brevets

(11) **EP 2 339 953 B1**

## (12) EUROPEAN PATENT SPECIFICATION

(45) Date of publication and mention
of the grant of the patent:
**10.01.2018 Bulletin 2018/02**

(21) Application number: **09764348.0**

(22) Date of filing: **26.10.2009**

(51) Int Cl.:
***A61B 5/145*** *(2006.01)*

(86) International application number:
**PCT/US2009/062011**

(87) International publication number:
**WO 2010/062535 (03.06.2010 Gazette 2010/22)**

(54) **METHODS FOR EVALUATING GLYCEMIC CONTROL**

VERFAHREN ZUR BEURTEILUNG DER GLYKÄMISCHEN KONTROLLE

PROCÉDÉS POUR ÉVALUER LE CONTRÔLE GLYCÉMIQUE

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **27.10.2008 US 108753 P
23.10.2009 US 604685**

(43) Date of publication of application:
**06.07.2011 Bulletin 2011/27**

(73) Proprietor: **Medtronic MiniMed, Inc.
Northridge, CA 91325 (US)**

(72) Inventor: **THOMAS, Andreas
01796 Pirna (DE)**

(74) Representative: **Ruschke, Hans Edvard
Ruschke Madgwick Seide & Kollegen
Postfach 86 06 29
81633 München (DE)**

(56) References cited:
**EP-A1- 1 788 540      EP-A1- 1 933 246
JP-A- 2004 164 343     JP-A- 2004 313 265
JP-A- 2006 043 360     US-B1- 6 322 516**

**Description**

FIELD OF THE INVENTION

Embodiments of the present invention are directed to methods for evaluating glycemic control of a patient. Specifically, the present disclosure provides an integrated description of glycemia in diabetic patients over a specific time interval, while including independent factors for assessing metabolic control.

BACKGROUND OF THE INVENTION

Self-monitoring blood glucose and measuring glycated hemoglobin ($HbA_{1c}$ or hemoglobin $A_{1c}$) values have become the established methods of assessing glycemic control of patients with diabetes. For these patients, who typically receive insulin treatments, the primary benefits of monitoring blood glucose levels 4 to 6 times a day are the ability to adapt their medication therapy themselves to their food intake and levels of physical activity, and to correct for non-physiological glycemic excursions. Therapists are particularly interested in the $HbA_{1c}$ value, because it helps them assess metabolic quality. Besides being easy to measure, this parameter is especially valuable because of the established correlation between protein glycosylation and the development of diabetic complications - a relationship that has been demonstrated in large clinical studies. This correlation has allowed the $HbA_{1c}$ value to gain acceptance as a target parameter in numerous national and international diabetes treatment guidelines. The simplicity in measuring and ease for a physician to interpret the $HbA_{1c}$ value has made it the standard parameter for the evaluation of glycemic control.

At the same time, the $HbA_{1c}$ value has been shown to correlate well with the mean glycemic level over the course of 8 to 12 weeks. This parameter, however, only represents part of the risk of disruptions in glucose homeostasis, namely the long-term profile; it does not describe acute fluctuations in blood and tissue glucose levels (i.e., glycemic variability) because glycosylated hemoglobin is present only in its labile aldimine state for the first six hours after formation. The stable ketoamine form only arises afterwards.

The significance of these glycemic variations is particularly clear with regard to the correlation between postprandial hyperglycemia and cardiovascular disorders, which was demonstrated as early as the 1990s in several studies. A further study performed during this time period shows that increased postprandial glucose excursions are associated with microvascular complications in patients with Type 2 diabetes. In-vitro studies performed on cells in which fluctuating glucose levels produce the greatest degree of oxidative stress, along with the highest rate of apoptosis, underscore the significance of glycemic variability. There are also indications that non-physiologically high postprandial excursions in patients with Type 2 diabetes are at the center of a cascade of diabetogenic and atherogenic events, such as increased insulin resistance, postprandial dyslipidemia, increased oxidative stress, a shift in the equilibrium in the coagulation cascade, endothelial dysfunction, etc. This problem is also relevant to patients with Type 1 diabetes. A Finnish study conducted over the course of 18 years was able to demonstrate that there are no significant differences between patients with Type 1 and Type 2 diabetes with respect to cardiovascular and overall mortality. To qualify these findings, however, it should be noted that no suitable prospective, randomized endpoint studies have been conducted to this point that prove a clear association between glycemic variations and microvascular/macrovascular events.

Hypoglycemic excursions, on the other hand, also contribute to an increase in glycemic variability. Associated with this is an adrenergic reaction that, at least in patients with existing vascular damage, increases the risk of severe complications, such as myocardial infarction and apoplectic stroke.

Continuous glucose monitoring (CGM) makes it possible to characterize a patient's glycemic profile in detail over the course of at least a few days. CGM systems have been available on the market since 1999 and are becoming increasingly accepted for diabetological diagnostics. CGM software works up data from recorded glucose profiles and calculates a variety of different parameters for glucose profile characterization; standardization is not yet a possibility, however. The following parameters for describing glycemic control have been suggested in the literature (in some cases in combination with each other):

- mean glucose concentration
- standard deviation for the mean glucose concentration
- the mean amplitude of glycemic excursions (MAGE), which describes the arithmetic mean of the difference between consecutive glycemic maxima and minima
- the number of hypoglycemic and hyperglycemic events
- the portion of each day spent in the hypoglycemic or hyperglycemic range
- the percentage of time spent each day in the euglycemic range
- mean of the maximum excursions in the hypoglycemic or hyperglycemic range
- CONGA (continuous overall net glycemic action)
- glucose lability index (LI)

- average daily risk range (ADRR), which encompasses both the low and high blood glucose indices (LBGI and HBGI)
- GRADE (glycemic risk assessment diabetes equation).

The essential difference between these parameters lies in the treatment of hypoglycemic excursions. MAGE and the standard deviation of the mean glucose level only take these into consideration indirectly, for instance, whereas ADRR and GRADE treat them directly. With the exceptions of MAGE and mean glucose concentration (indirectly via the relationship to $HbA_{1c}$), these various parameters have not been evaluated with respect to the quality of metabolic control and the risk of developing complications of diabetes. It follows that no verifiable conclusions may be drawn at the present time regarding the relationship between parameters such as these, which describe acute glycemia, and the $HbA_{1c}$ value, which describes long-term metabolic control.

Despite the availability of analysis software, a detailed assessment of glucose profiles would be somewhat time consuming. This reason, along with other reasons (such as cost), constitutes an important reason why practical application of CGM has been relatively infrequent to date. In other words, it is difficult to obtain a quick overview from these measurements and to reach conclusions for the prognosis of diabetic complications. As such, quickly filtering core parameters from recorded glucose profiles and making them available in such a way that they may be applied and interpreted at a glance for a rapid assessment of a patient's glycemic profile would be an extremely worthwhile project.

It would be advantageous to have a simple, straightforward model based on various parameters that are either available from glucose profiles or that may be calculated quickly from profiles. It is desirable to create a model that yields a value that characterizes the course of acute and long-term glycemia.

JP 2004313265 A discloses a health management method and health management system. It provides an incentive for performing health ameliorating actions to a user in order to reduce the dropping out of the user in an initial stage for performing the health ameliorating actions. The health state of the individual user is comprehended by performing a questionnaire investigation and the measurement of biological data. After a prescribed period of time has passed since the provision of the advice, a predetermined consciousness related to the healthy action is evaluated for the user, and the evaluation result is indicated to the user.

JP 2004164343 A discloses a life-style related disease ameliorate system and an information management apparatus for the same to understand a content of hearing from a patient in a short time and explaining an output result to the patient in a clearly understandable way by focusing only on minimum necessary items and making expression to be intuitively and clearly understood. The system includes an input means for inputting information on eating habits of the patient, information on living habits of the patient and/or information on the result of medical checkup. An accumulation means for accumulating the input information in a chronological order for each patient and an output means for outputting the accumulated time-series information for each patient in a way that the time-series changes of the information can be recognized.

JP 2006043360 discloses a method for grasping whole picture in quality and quantity of fat. It supports health management by providing the whole situation of fat in a form to allow not an expert but a patient or a physical examination receiver to understand it. The inspection result of the quality and quantity of the fat is expressed at a time by a radar chart. Accordingly, the whole situation of the fat is made to be understood so as to synthetically grasp the risk of arteriosclerosis or myocardial infarction at a glance.

SUMMARY OF THE INVENTION

According to the present invention there is provided a method as defined in claim 1. Preferred embodiments are defined in the dependent claims. According to the present disclosure, there is provided a method of evaluating glycemic control of a patient includes providing a pentagon having five axes radiating from a center of the pentagon. A first axis has a length representing a range of hemoglobin $A_{1c}$ values. A second axis has a length representing a range of standard deviation of glucose values. A third axis has a length representing a range of amount of time per day values exceeding a first limit. A fourth axis has a length representing a range of daily area-under-curve values exceeding a second limit. A fifth axis has a length representing a range of mean glucose values. A first point on the first axis indicative of a representative hemoglobin $A_{1c}$ value is plotted. A second point on the second axis indicative of a representative standard deviation of glucose value is plotted. A third point on the third axis indicative of a representative amount of time per day value exceeding the first limit is plotted. A fourth point on the fourth axis indicative of a representative daily area-under-curve value exceeding a second limit is plotted. A fifth point on the fifth axis indicative of a representative mean glucose value is plotted. A sixth point on the first axis indicative of a hemoglobin $A_{1c}$ value of the patient is plotted. A seventh point on the second axis indicative of a standard deviation of glucose value of the patient is plotted. An eight point on the third axis indicative of an amount of time per day value exceeding the first limit of the patient is plotted. A ninth point on the fourth axis indicative of a daily area-under-curve value exceeding the second limit of the patient is plotted. A tenth point on the fifth axis indicative of a mean glucose value of the patient is plotted. A first pentagon area formed by the first point, the second point, the third point, the fourth point, and the fifth point is determined. A second pentagon area formed by the sixth point, the seventh point, the eighth point, the ninth point, and the tenth point is determined. A glycemic control parameter is determined based on the first pentagon area and the second pentagon area.

The glycemic control parameter is determined by dividing the second pentagon area by the first pentagon area. The representative hemoglobin $A_{1c}$ value, the representative standard deviation of glucose value, the representative amount of time per day value exceeding the first limit, the representative daily area-under-curve value exceeding the second limit, and the representative mean glucose value may be representative of a non-diabetic individual. The first limit may be 160 mg/dL. The second limit may be 160 mg/dL. The method may be implemented on a computing device. The method may be implemented on an infusion device. The method may be implemented on an infusion device controller/programmer. The method may be implemented on a medical device. The first axis representing the range of hemoglobin $A_{1c}$ values and the fifth axis representing the range of mean glucose values are adjacent to each other in the pentagon.

The present disclosure provides also an article of manufacture containing code for evaluating glycemic control of a patient, comprising a computer-usable medium including at least one embedded computer program that is capable of causing at least one computer to perform providing a pentagon having five axes radiating from a center of the pentagon. A first axis has a length representing a range of hemoglobin $A_{1c}$ values. A second axis has a length representing a range of standard deviation of glucose values. A third axis has a length representing a range of amount of time per day values exceeding a first limit. A fourth axis has a length representing a range of daily area-under-curve values exceeding a second limit. A fifth axis has a length representing a range of mean glucose values. A first point on the first axis indicative of a representative hemoglobin $A_{1c}$ value is plotted. A second point on the second axis indicative of a representative standard deviation of glucose value is plotted. A third point on the third axis indicative of a representative amount of time per day value exceeding the first limit is plotted. A fourth point on the fourth axis indicative of a representative daily area-under-curve value exceeding a second limit is plotted. A fifth point on the fifth axis indicative of a representative mean glucose value is plotted. A sixth point on the first axis indicative of a hemoglobin $A_{1c}$ value of the patient is plotted. A seventh point on the second axis indicative of a standard deviation of glucose value of the patient is plotted. An eight point on the third axis indicative of an amount of time per day value exceeding the first limit of the patient is plotted. A ninth point on the fourth axis indicative of a daily area-under-curve value exceeding the second limit of the patient is plotted. A tenth point on the fifth axis indicative of a mean glucose value of the patient is plotted. A first pentagon area formed by the first point, the second point, the third point, the fourth point, and the fifth point is determined. A second pentagon area formed by the sixth point, the seventh point, the eighth point, the ninth point, and the tenth point is determined. A glycemic control parameter is determined based on the first pentagon area and the second pentagon area.

The glycemic control parameter is determined by dividing the second pentagon area by the first pentagon area. The representative hemoglobin $A_{1c}$ value, the representative standard deviation of glucose value, the representative amount of time per day value exceeding the first limit, the representative daily area-under-curve value exceeding the second limit, and the representative mean glucose value may be representative of a non-diabetic individual. The first limit may be 160 mg/dL. The second limit may be 160 mg/dL. The article may be a computing device. The article may be an infusion device. The article may be an infusion device controller/programmer. The article may be a medical device. The first axis representing the range of hemoglobin $A_{1c}$ values and the fifth axis representing the range of mean glucose values are adjacent to each other in the pentagon.

## BRIEF DESCRIPTION OF THE DRAWINGS

FIG. 1 illustrates a Glucose Pentagon according to embodiments of the present invention.

FIG. 2 illustrates a Glucose Pentagon comparing a pentagon of a non-diabetic person to a pentagon of a diabetic patient according to embodiments of the present invention.

FIG. 3 illustrates a representative relationship between a Glycemic Risk Parameter (GRP) value and the risk of developing diabetic complications according to embodiments of the present invention.

FIGS. 4A-4C illustrate sample continuous glucose monitoring (CGM) profiles over three days and the resultant Glucose Pentagons for a representative diabetic patient according to embodiments of the present invention.

FIGS. 5A-5D illustrate Glucose Pentagons for a selected day for a plurality of diabetic patients.

FIG. 6 illustrates a flow chart of evaluating glycemic control of a patient according to embodiments of the present invention.

## DETAILED DESCRIPTION

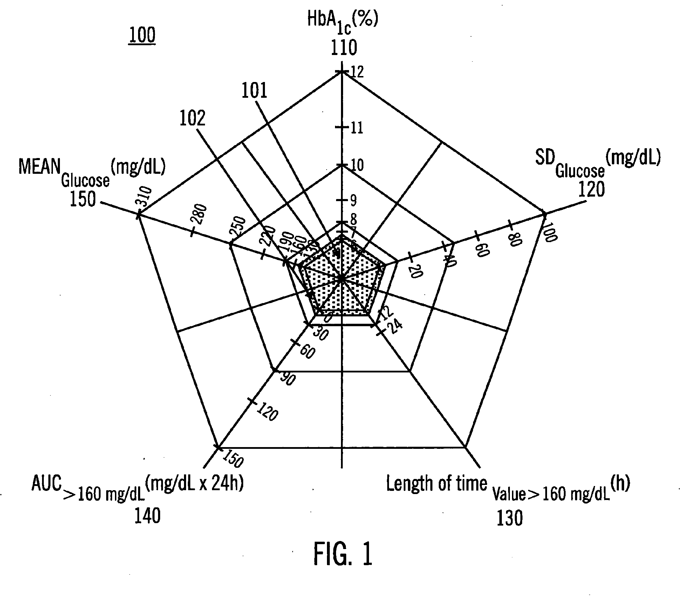

FIG. 1 illustrates a Glucose Pentagon according to embodiments of the present invention. Five parameters are calculated from glucose profiles of diabetic and non-diabetic (healthy) patients, and each parameter forms a single axis of a five-sided figure, the Glucose Pentagon 100:

- the $HbA_{1c}$ value (this is not calculated, but is instead incorporated as an existing value) axis 110
- the standard deviation of the mean glucose concentration axis 120
- the amount of time per day in hyperglycemic values (e.g., >160 mg/dL = 8.9 mmol/L) axis 130
- the area-under-curve (AUC) of hyperglycemic values (e.g., >160 mg/dL = 8.9 mmol/L) axis 140
- the mean glucose concentration axis 150.

Taken together, the selected parameters provide an integrated description of glycemia over the period of time under observation. These parameters also make it possible to incorporate other data indirectly, such as, for example, preprandial glycemia, postprandial glycemic excursions, and MAGE, whereby the mean glucose concentration describes the average glycemic situation and the standard deviation describes glycemic variability to a certain degree:

Using MAGE as a parameter for the Glucose Pentagon 100 instead of the standard deviation also may be an option, as this better characterizes extreme glycemic excursions. Measuring oxidative stress by determining the rate at which 8-iso $PGF_{2\alpha}$ is excreted in the urine yields a MAGE value of 45 mg/dL in individuals with healthy metabolism. Values up to approximately 150 mg/dL (mean: 75 mg/dL) have been recorded in patients with Type 2 diabetes; this value may range up to approximately 280 mg/dL (mean: 140 mg/dL) in patients with Type 1 diabetes. No definitive correlation has been demonstrated between this marker and MAGE (r = - 0.381). Hence, MAGE was not taken into consideration in the Glucose Pentagon 100 of patients with Type 1 diabetes, although it may be still utilized in alternative embodiments.

Including the $HbA_{1c}$ value in the Glucose Pentagon 100 links the parameters determined from glucose profiles with what is recognized as the best parameter for characterizing long-term metabolic control. It is true that, to a large extent, a linear correlation (r = 0.876) between the $HbA_{1c}$ value and the mean glycemic value determined from continuous glucose monitoring (CGM) entries does exist. This correlation may be defined, for example, by the following equation:

$$\text{mean glucose }_{(CGM \text{ over 3 months})} \text{ [mmol/L]} = 1.649 \times HbA_{1c} - 2.645$$

As such, this value is theoretically already represented in the Glucose Pentagon 100. If the information provided by the mean glucose concentration is to be as meaningful as that yielded by the $HbA_{1c}$ value, however, the glucose profile must not contain any relatively long gaps over the 3-month time period under consideration. This issue has almost always been the case with day-to-day monitoring, however, at least up to now, which is why the $HbA_{1c}$ value was incorporated into the Glucose Pentagon 100. Another advantage of integrating the $HbA_{1c}$ value is that it provides a link to a verified laboratory diagnostic value covering a glycemic period of 8-10 weeks.

The time per day values and area-under-curve (AUC) per day values at blood glucose levels of, for example but not necessarily limited to, greater than 160 mg/dL are both parameters that characterize hyperglycemic phases over the course of a day, and are considered to be additional risk parameters for developing diabetic complications. The daily time AUC value clearly correlates with oxidative stress and, as such, is relevant to the development of vascular complications. These parameters are assigned their own independent significance, as both are only partially reflected in the calculated mean/standard deviation and the $HbA_{1c}$ value. A value of 160 mg/dL is taken, for example, as the threshold value for normal glycemia and thus increased risk. This value was selected because it represents a typical, postprandial maximum value for individuals with healthy metabolism whose glucose profiles are recorded with CGM, although according to alternative embodiments, any other suitable value may be utilized.

Time and AUC in the hypoglycemic range are not taken into consideration directly, however, as these values do not correlate directly with the risk of developing diabetic complications. The controversial influence of hypoglycemic events on mortality rate may be surmised and should be primarily interpreted as an acute event in patients with existing vascular damage. Due to subsequent autonomic counter-regulation, however, hypoglycemic events do have an indirect impact on glycemic variability. This effect is encompassed by the standard deviation of the mean glucose concentration. In principle, rates of hypoglycemia, time of hypoglycemic events and AUC represent a trio of parameters lying outside the Glucose Pentagon 100.

The values taken into consideration here cover a surface area that is easy to calculate and that may be viewed as an independent, integrated parameter for describing glycemia. A meaningful way of obtaining a dimensionless value is to normalize this area using the following values recorded in CGM profiles of individuals with healthy metabolism. The resulting area is shown as inner pentagon 101 in FIG. 1. The values forming the inner pentagon 101 are as follows:

- $HbA_{1c}$ value: $\leq 5.5\%$
- standard deviation of the glucose concentration: $\pm$ 10 mg/dL (0.55 mmol/L)

- time per day >160 mg/dL (8.9 mmol/L): 0 min
- AUC > 160 mg/dL (8.9 mmol/L): 0 mg/dL x day
- mean glucose concentration: 90 mg/dL (5 mmol/L)

The area calculated for the glucose pentagon of a patient with diabetes, divided by the reference/standard pentagon area 101 of healthy individuals, provides a more meaningful assessment of a patient's risk of developing diabetic complications than is possible with just the $HbA_{1c}$ value. The reason for this conclusion is that the Glucose Pentagon 100 incorporates parameters providing information on glycemic variability. This feature is not the case with $HbA_{1c}$ alone.

The starting point for the axes 110, 120, 130, 140, 150 are determined using values from healthy individuals, whereby even in these cases values lie above zero. The influence of individual parameters on the risk of developing microvascular and macrovascular complications must be taken into consideration when selecting the scale of the axes. With respect to the $HbA_{1c}$ value, studies have established this influence for patients with Type 1 and Type 2 diabetes. Risk curves for developing complications do not indicate the same degree of risk for microalbuminuria, neuropathy, nephropathy and retinopathy (Type 1 diabetes) and/or for microvascular or macrovascular end points (Type 2 diabetes). As such, a reasonable approach would be to define an average function that is based on these curves and dependent on the $HbA_{1c}$ value. Theoretically, however, the Glucose Pentagon 100 also may be calculated specifically for each individual complication.

The mean glucose concentration is closely correlated to the $HbA_{1c}$ value. The scale for the daily time AUC value in the hyperglycemic range, in turn, is oriented toward this mean glucose value, in that the threshold for hyperglycemia (160 mg/dL = 8.9 mmol/L) is subtracted from each mean glucose value. Establishing the scale for the two other parameters is more difficult. No clinical study data on time spent in the hyperglycemic range is currently available. Reference therefore only may be made to studies on the rate of apoptosis in human umbilical endothelial cells under conditions of continuous and variable glycemia, whereby the relationship is presumably linear. We have likewise assumed a linear scale for the standard deviation value of the mean glucose concentration - an assumption based on various studies on the relationship between oxidative stress markers and glucose variability.

Of critical concern is the ability to estimate which errors will arise in the overall Glucose Pentagon 100 when individual parameters vary. Unlike the $HbA_{1c}$ value, which yields virtually no information on glycemic variability when taken alone, the area of the Glucose Pentagon 100 provides a more extensive and better description. Because the $HbA_{1c}$ value is entered into the model as a constant that does not change until the next measurement is taken, an "error" arises when this parameter briefly improves or worsens relative to its baseline. The maximum error caused by such a situation may be, however, estimated using the correlation between the mean glucose value and the $HbA_{1c}$ value. The estimated error may be indicated for the $HbA_{1c}$ value in the Glucose Pentagon 100 at any given point in time as $\Delta F_{HbA1c}$, which represents the deviation of the current (but not of the most recently measured) $HbA_{1c}$ value. This fact is immediately apparent in the Glucose Pentagon 100: the line connecting the axes for $HbA_{1c}$ 110 and mean glucose 150 runs parallel to the edge of the standard pentagon area 101 if the mean glucose corresponds to the $HbA_{1c}$ value. If the mean glucose value is "better" than the $HbA_{1c}$ value, then the connecting line will be angled toward the center of the Glucose Pentagon 100 at the point where it meets the $MEAN_{Glucose}$ axis 150; if the value is worse, the line will angle outwards.

Measurement errors that occur during the process of recording the glucose profile, or during the process of determining the $HbA_{1c}$ value, also give rise to discrepancies between the mean glucose concentration and the $HbA_{1c}$ value. "Errors" in $HbA_{1c}$ measurements also may have pathological sources. Hemoglobinopathies or hemolytic anemia, for instance, yield false low values, whereas chronic iron deficiency anemia causes false high values for $HbA_{1c}$. Discrepancies of this type are immediately apparent in the Glucose Pentagon 100. This issue also may be confirmed by dividing the mean glucose concentration by the $HbA_{1c}$ value - a concept similar to the Glyc-Q parameter, a value which is obtained through the division of fructosamine by $HbA_{1c}$ (Glyc-Q = Fructosamine x 2.2 / $HbA_{1c}$).

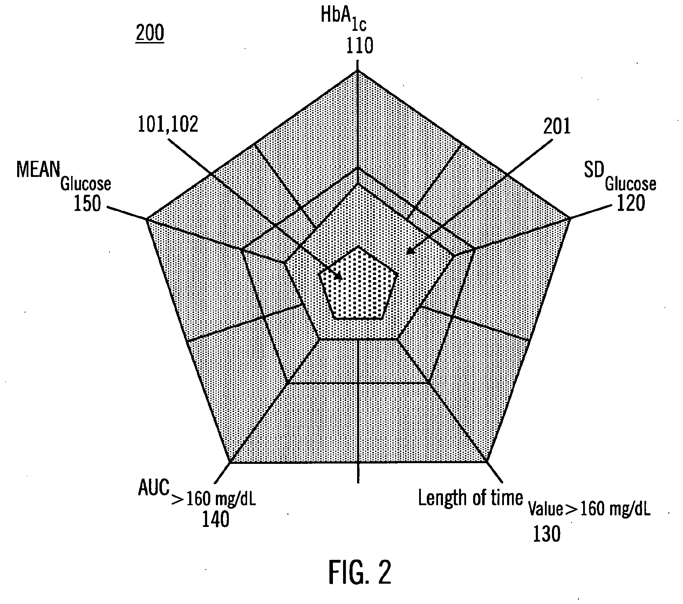

FIG. 2 illustrates a Glucose Pentagon comparing a pentagon of a non-diabetic person to a pentagon of a diabetic patient. Taking the area of the glucose pentagon 201 for a diabetic patient, plotted utilizing five points on the five axes 110, 120, 130, 140, 150, respectively, indicative of the diabetic patient's $HbA_{1c}$ and CGM values, and normalizing it to the standard area of the glucose pentagon 101 for a healthy/non-diabetic individual, plotted utilizing five points on the five axes 110, 120, 130, 140, 150, respectively, indicative of a healthy/non-diabetic person's $HbA_{1c}$ and CGM values (or alternatively, normalizing it to the standard area of the glucose pentagon 102, as illustrated in FIGS. 1 and 2, representing the range in which the risk of diabetes patients developing diabetic complications is low) yields a non-dimensional characteristic value defined as the Glycemic Control Parameter or Glycemic Risk Parameter (GRP):

$$GRP = \frac{\text{area of the glucose pentagon of a diabetic patient}}{\text{area of the glucose pentagon for healthy/non-diabetic individual}}$$

This parameter quickly allows an assessment of a patient's metabolic control while taking significantly more factors into consideration than is possible by looking solely at the $HbA_{1c}$ value. The GRP may be established as a relatively easily determined parameter that better describes an individual patient's risk of developing diabetic complications. A scale that offers a rapid overview of metabolic conditions on each individual day then may be developed in parallel. When viewed in their entirety over time, numerous values such as these will yield information comparable to the $HbA_{1c}$ value because they incorporate data on acute and long-term glycemia; however such values will provide a significantly more comprehensive picture of the situation. Unlike the $HbA_{1c}$ value, however, the GRP for a given time period is available at any time. Furthermore, the parameters integrated within the GRP also may be considered separately when a more detailed glycemic assessment is required.

Metabolic control may be subsequently assessed on two fundamental levels:

- the GRP as an integrated parameter for assessing glycemia and as a parameter for monitoring daily success (risk control)
- the individual parameters of mean, standard deviation, AUC, hyperglycemic time, and $HbA_{1c}$ for a detailed glycemic assessment.

In practice, a suitable software system may be utilized as a simple means of determining the GRP and the individual parameters of the Glucose Pentagon 100 from a measured CGM profile. Such a system would not only calculate the values of the various parameters, but would also plot the corresponding chart and determine the GRP. Software integrated into CGM systems may be reprogrammed accordingly. The only required input is the most up-to-date $HbA_{1c}$ value available.

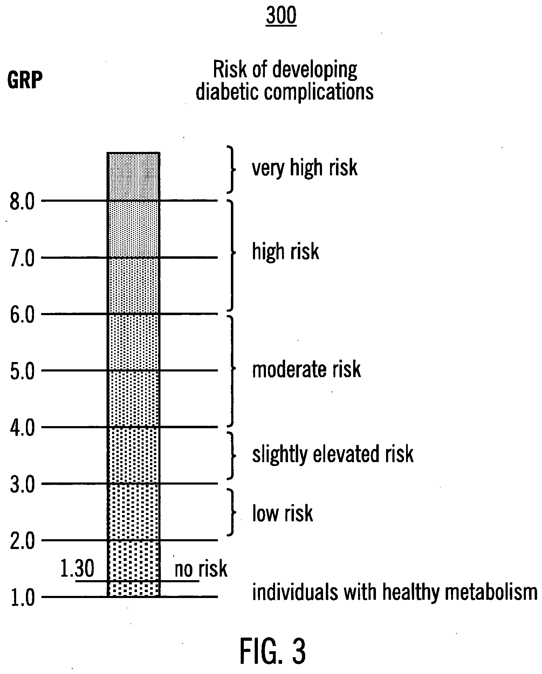

FIG. 3 illustrates a representative relationship between a Glycemic Risk Parameter (GRP) value and the risk of developing diabetic complications. A graphic representation 300 of the calculated GRP, which may be color-coded according to the risk of developing diabetic complications, may allow health professionals and patients to directly gauge the success of their efforts in order to optimize metabolic control. At the same time, concrete data and the other parameters underlying the Glucose Pentagon 100 may be available to the therapist for a more detailed analysis.

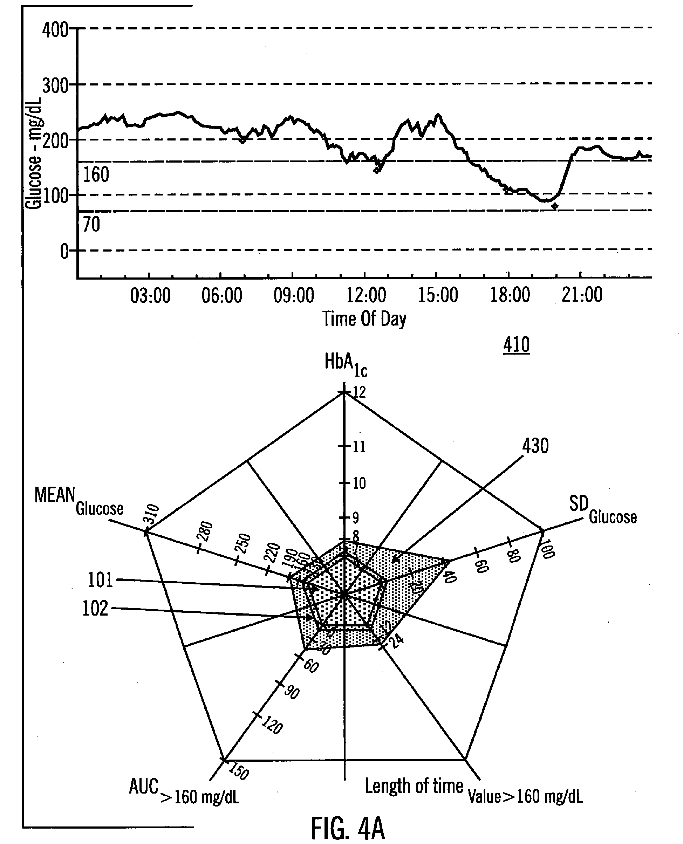

FIGS. 4A-4C illustrate sample continuous glucose monitoring (CGM) profiles over three days and the resultant Glucose Pentagons for a representative diabetic patient. The following examples according to embodiments of the present invention use data from patients with Type 1 diabetes and are intended to illustrate how the Glucose Pentagon 100 may be used in practice. The patient's CGM data is represented in graphs 410, 440, 470 for Day 1, Day 2, and Day 3, respectively. An $HbA_{1c}$ value of 7.5% had most recently been measured for a 49-year-old female patient with Type 1 diabetes who had suffered from diabetes for 40 years and managed her condition with insulin pump therapy combined with a rapid-acting analog insulin. Blood pressure and lipid parameters were well regulated with a beta-blocker, an ACE inhibitor, and a statin. Known conditions included retinopathy, nephropathy, peripheral neuropathy and stage 2 peripheral arterial occlusive disease (PAOD).

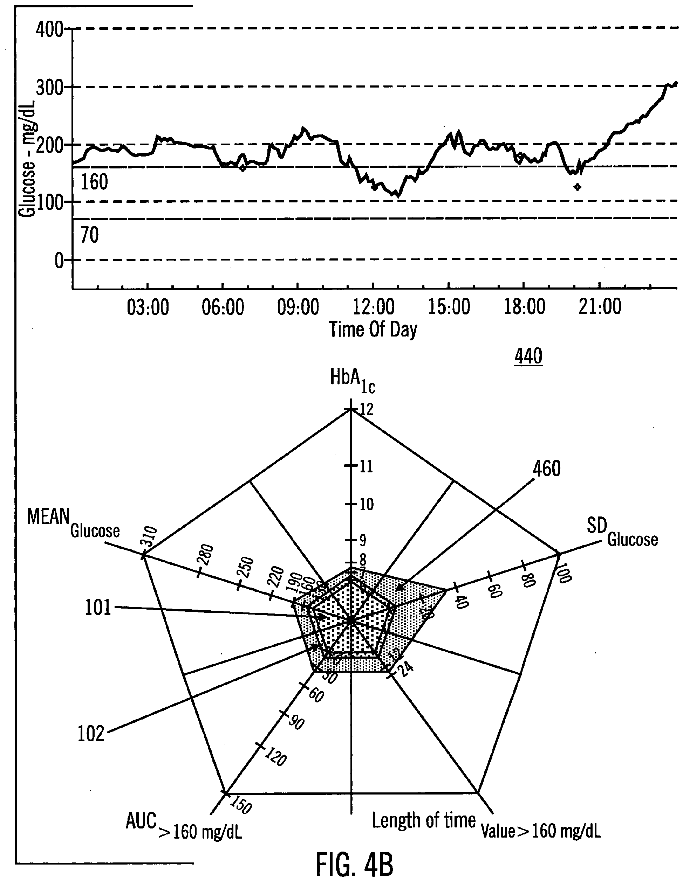

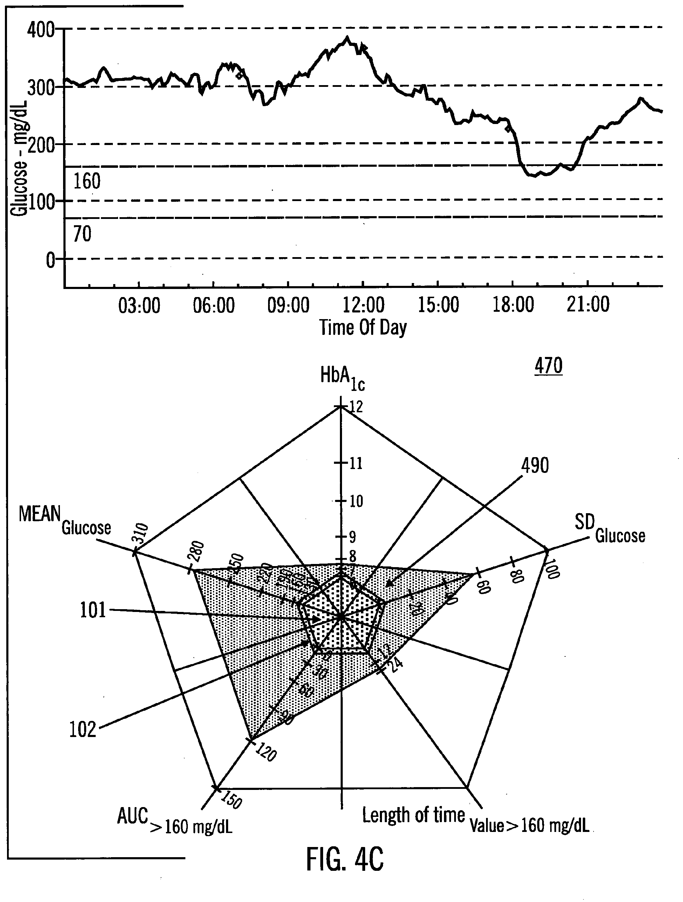

The underlying parameters and the resulting GRP are given in the following table (referring to FIGS. 4A, 4B, and 4C corresponding to Day 1, Day 2, and Day 3, respectively):

|  | Day 1 | Day 2 | Day 3 |
|---|---|---|---|
| $HbA_{1c}$ (%) | 7.5 | 7.5 | 7.5 |
| $MEAN_{glucose}$ (mg/dL) | 191 | 188 | 278 |
| $SD_{glucose}$ (mg/dL) | 44 | 34 | 57 |
| $AUC_{> 160\,mg/dL}$ (mg/dL x day) | 39 | 32 | 118 |
| $Time/da_{> 160\,mg/dL}$ (min.) | 1155 | 1230 | 1325 |
| GRP | 3.30 | 2.87 | 7.38 |

The average GRP from these three days, calculated from the glucose pentagons 430, 460, 490, relative to the reference non-diabetic/healthy glucose pentagon 101 (or 102), as illustrated in FIGS. 4A-4C, is 4.52, which indicates an increased risk of diabetic complications (referring to FIG. 3). One suspects that these values are typical for the patient, as clearly evidenced by the existing diabetic complications. The pattern in the glucose pentagons 430, 460, 490 also shows excursions toward high glucose variability on all days (standard deviation of the mean glucose concentration). Another noticeable characteristic is that the mean glucose concentration is higher than the current, most recently measured $HbA_{1c}$ value for all three days. If these values represent the trend over a relatively long period of time, one might anticipate that the subsequent $HbA_{1c}$ value will have worsened.

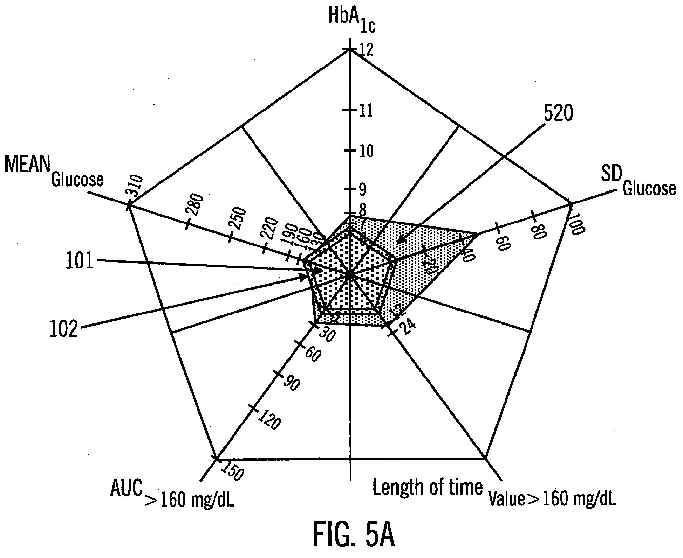

FIGS. 5A-5D illustrate Glucose Pentagons for a selected day for a plurality of diabetic patients. Referring to Table 1 below, CGM data is collected for three days for three representative diabetic patients, and their resulting GRP values and average three-day GRP values are calculated using the Glucose Pentagon 100. Patient JE includes data with analog insulin (a), and normal insulin (b), as indicated in Table 1 below.

TABLE 1

| Patient | Data for calculations | Day 1 | Day 2 | Day 3 | Avg. GRP (3 days) |
|---|---|---|---|---|---|
| JE, male, T1D, CSII | $HbA_{1c}$ (%) | 7.7 | | | |
| Age: 40 | $MEAN_{glucose}$ (mg/dL) | 115 | 111 | 128 | |
| Years with diabetes: 10, no complications | $SD_{glucose}$ (mg/dL) | 65 | 47 | 49 | |
| a) with analog insulin | $AUC_{> 160\,mg/dL}$ (mg/dL x day) | 14 | 25 | 9 | |
| b) with normal insulin | $Time/day_{> 160\,mg/dL}$ (min.) | 305 | 865 | 270 | |
| | GRP | 2.99 | 2.82 | 2.62 | 2.81 |
| | $HbA_{1c}$ (%) | | | 7.7 | |
| | $MEAN_{glucose}$ (mg/dL) | 153 | 200 | 175 | |
| | $SD_{glucose}$ (mg/d L) | 59 | 79 | 62 | |
| | $AUC_{> 160\,mg/dL}$ (mg/dL x day) | 22 | 52 | 33 | |
| | $Time/day_{> 160\,mg/dL}$ (min.) | 630 | 980 | 870 | |
| | GRP | 3.07 | 4.22 | 3.52 | 3.60 |
| | | | | | |
| HB, male, T1D, ICT | $HbA_{1c}$ (%) | | | 6.2 | |
| Age: 60 | $MEAN_{glucose}$ (mg/dL) | 124 | 119 | 164 | |
| Years with diabetes: 36, complications: retinopathy, nephropathy, neuropathy Analog insulins | $SD_{glucose}$ (mg/dL) | 35 | 41 | 37 | |
| | $AUC_{> 160\,mg/dL}$ (mg/dL x day) | 2 | 4 | 17 | |
| | $Time/day_{> 160\,mg/dL}$ (min.) | 315 | 255 | 770 | |
| | GRP | 2.10 | 2.03 | 2.20 | 2.11 |
| | | | | | |
| CT, female, T1D, ICT | $HbA_{1c}$ (%) | 11.3 | | | |
| Age: 17 | $MEAN_{glucose}$ (mg/dL) | 274 | 255 | 262 | |

(continued)

| Patient | Data for calculations | Day 1 | Day 2 | Day 3 | Avg. GRP (3 days) |
|---|---|---|---|---|---|
| Years with diabetes: 4, | $SD_{glucose}$ (mg/dL) | 55 | 38 | 70 | |
| complications: neuropathy | $AUC_{>160\ mg/dL}$ (mg/dL x day) | 94 | 81 | 75 | |
| Normal insulin/NPH | $Time/day_{>160\ mg/dL}$ (min.) | 1340 | 1250 | 1280 | |
| insulin | GRP | 10.59 | 8.41 | 9.97 | 9.66 |

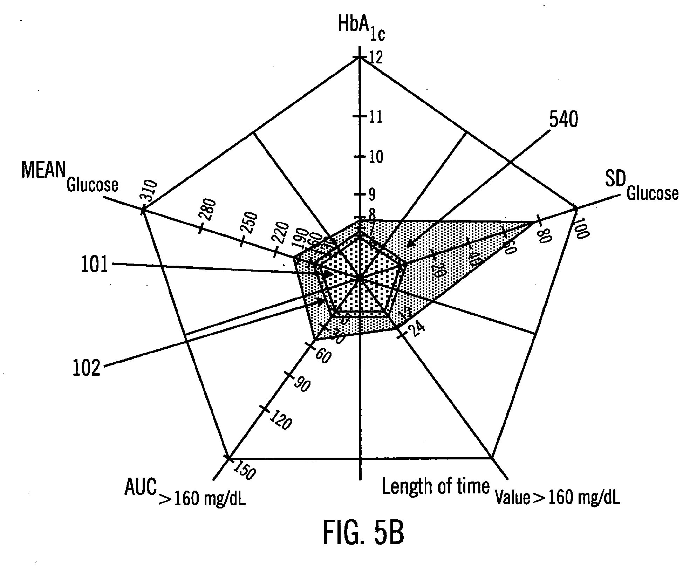

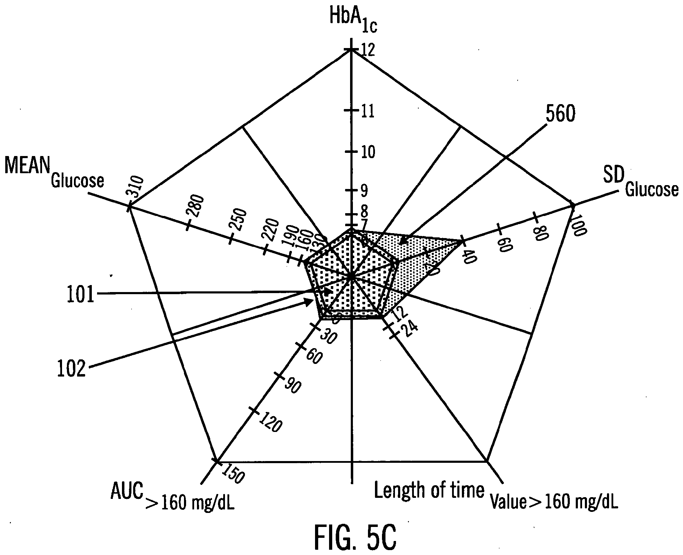

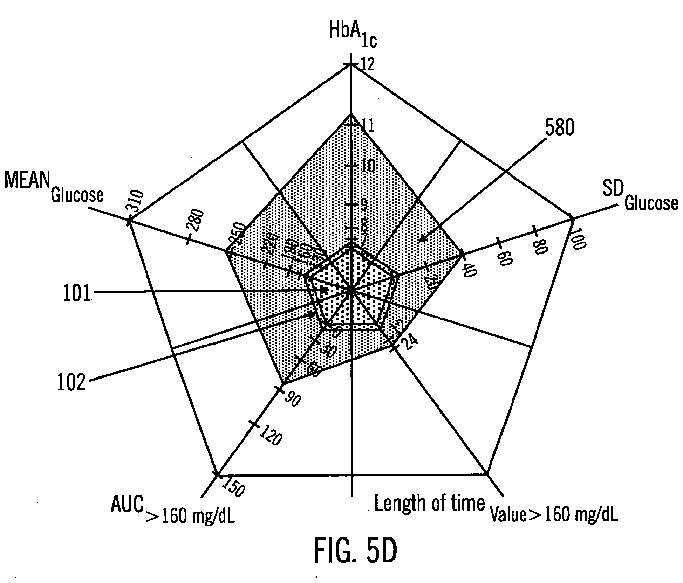

FIGS. 5A-5D illustrate the corresponding glucose pentagons on one day selected for each patient in Table 1 above. The glucose pentagons 520, 540, 560 for the first two patients (JE in FIGS. 5A and 5B, and HB in FIG. 5C) are characterized predominantly by glycemic variability, whereas the high $HbA_{1c}$ value and mean glucose concentration are responsible for the large pentagon 580 for patient CT in FIG. 5D. The comparison between the use of normal insulin (FIG. 5B) and rapid-acting analog insulin (FIG. 5A) in patient JE shows reduced glycemic variability with the analog insulin, resulting in better metabolic control. This kind of analysis is not possible when taking only the $HbA_{1c}$ value into consideration and demonstrates the sense in combining long-term and acute glycemia within the GRP parameter.

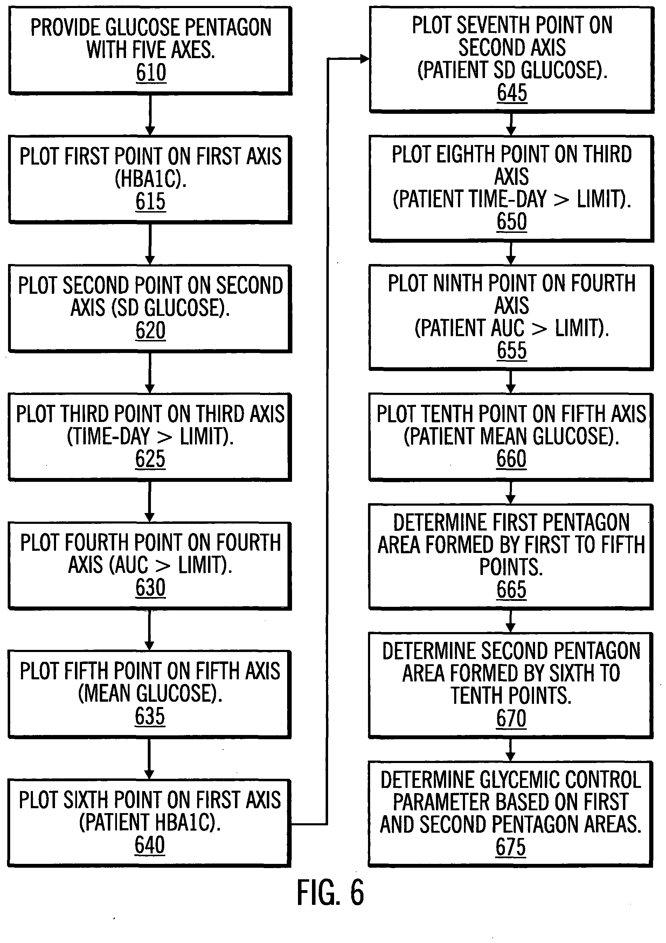

FIG. 6 illustrates a flow chart of evaluating glycemic control of a patient according to embodiments of the present invention. At step 610, a pentagon having five axes radiating from a center of the pentagon (see, e.g., Glucose Pentagon 100, FIG. 1) is provided. A first axis 110 (FIG. 1) has a length representing a range of hemoglobin $A_{1c}$ values. A second axis 120 (FIG. 1) has a length representing a range of standard deviation of glucose values. A third axis 130 (FIG. 1) has a length representing a range of amount of time per day values exceeding a first limit. A fourth axis 140 (FIG. 1) has a length representing a range of daily area-under-curve values exceeding a second limit. A fifth axis 150 (FIG. 1) has a length representing a range of mean glucose values. According to embodiments of the present invention, the first axis representing the range of hemoglobin $A_{1c}$ values and the fifth axis representing the range of mean glucose values may be adjacent to each other in the pentagon.

At step 615, a first point on the first axis 110 (FIG. 1) indicative of a representative hemoglobin $A_{1c}$ value is plotted. At step 620, a second point on the second axis 120 (FIG. 1) indicative of a representative standard deviation of glucose value is plotted. At step 625, a third point on the third axis 130 (FIG, 1) indicative of a representative amount of time per day value exceeding the first limit is plotted. According to embodiments of the present invention, the first limit may be 160 mg/dL. At step 630, a fourth point on the fourth axis 140 (FIG. 1) indicative of a representative daily area-under-curve value exceeding a second limit is plotted. According to embodiments of the present invention, the second limit may be 160 mg/dL. At step 635, a fifth point on the fifth axis 150 (FIG. 1) indicative of a representative mean glucose value is plotted.

At step 640, a sixth point on the first axis 110 (FIG. 1) indicative of a hemoglobin $A_{1c}$ value of the patient is plotted. At step 645, a seventh point on the second axis 120 (FIG. 1) indicative of a standard deviation of glucose value of the patient is plotted. At step 650, an eight point on the third axis 130 (FIG. 1) indicative of an amount of time per day value exceeding the first limit of the patient is plotted. At step 655, a ninth point on the fourth axis 140 (FIG. 1) indicative of a daily area-under-curve value exceeding the second limit of the patient is plotted. At step 660, a tenth point on the fifth axis 150 (FIG. 1) indicative of a mean glucose value of the patient is plotted.

A first pentagon area (see, e.g., pentagon 101 in FIG. 2) formed by the first point, the second point, the third point, the fourth point, and the fifth point on axes 110, 120, 130, 140, 150, respectively, is determined at step 665. A second pentagon area (see, e.g., pentagon 201 in FIG. 2) formed by the sixth point, the seventh point, the eighth point, the ninth point, and the tenth point on axes 110, 120, 130, 140, 150, respectively, is determined at step 670. At step 675, a glycemic control parameter (or Glycemic Risk Parameter - GRP) is determined based on the first pentagon area 101 and the second pentagon area 201. According to embodiments of the present invention, the glycemic control parameter (or Glycemic Risk Parameter - GRP) is determined by dividing the second pentagon area 201 (FIG. 2) by the first pentagon area 101 (FIG. 1).

According to embodiments of the present invention, the representative hemoglobin $A_{1c}$ value, the representative standard deviation of glucose value, the representative amount of time per day value exceeding the first limit, the representative daily area-under-curve value exceeding the second limit, and the representative mean glucose value plotted to determine the first pentagon area 101 (FIGS. 1 and 2) are representative of a non-diabetic/healthy individual.

The evaluation of glycemic control of a patient according to embodiments of the present invention may be implemented on a computing device such as a computer system (e.g., desktop, laptop, enterprise systems, network/Web systems, etc.), a handheld device (e.g., PDAs), a mobile/smart phone, a medical device, an infusion device (e.g., insulin pumps), an infusion device controller/programmer, a hospital monitor, or any other suitable electronic device. Moreover, an article of manufacture (such as, e.g., a memory storage device such as a RAM/ROM, optical disk, flash memory, hard disk drive, etc., a computing device such as a computer system (e.g., desktop, laptop, enterprise systems, network/Web systems, etc.), a handheld device (e.g., PDAs), a mobile/smart phone, a medical device, an infusion device (e.g., insulin pumps), an infusion device controller/programmer, a hospital monitor, or any other suitable electronic device) containing code for evaluating glycemic control of a patient as discussed above, comprising a computer-usable medium including at least one embedded computer program that is capable of causing at least one computer to perform the evaluation of glycemic control of a patient as discussed above according to embodiments of the present invention, also may be utilized.

The Glucose Pentagon 100 provides an integrated description of glycemia in diabetic patients over a specific time interval, while it also includes independent factors for assessing metabolic control. The time interval may be even just a single day, which is a useful feature. The $HbA_{1c}$ value is the only parameter that remains constant until it is measured again, but because it typically changes very little during short time intervals, the resulting error may be assumed to be negligible. This error does increase, however, the older the $HbA_{1c}$ measurement is and the more the value changes. The Glucose Pentagon 100 is much less subject to error, however, than an assessment of metabolic control based solely on the $HbA_{1c}$ value.

It would presumably make sense to determine the GRP for each individual day according to embodiments of the present invention so that the patient may use the pentagon to assess their day-to-day efforts. A mean GRP value then may be calculated over longer periods of time.

One advantage of embodiments of the present invention is that it takes both long-term and acute metabolic control into account, i.e., it unites $HbA_{1c}$ and glycemic fluctuations in a single model. Because it yields a characteristic numerical value, the GRP serves as a good starting point for assessing the risk of developing diabetic complications and provides far more information than the $HbA_{1c}$ value on its own. Detailed insight may be derived from the shape of the pentagon, which provides a quick overview of a patient's daily routine without having to look at the statistical details of the CGM profile. It also serves as a reference point for long-term care and clinical research. For the model to be useful, CGM software may perform glucose pentagon calculations and provide an opportunity for entering the $HbA_{1c}$ value.

Specialized glucose pentagons also may be determined in addition to the integrated glucose pentagon. These embodiments likewise may be incorporated into the CGM software, providing information on various types of diabetic complications, such as retinopathy, neuropathy, nephropathy, and cardiovascular events. It may be helpful to also distinguish between glucose pentagons for patients with Type 1 diabetes and those for individuals with Type 2 diabetes.

The inclusion of additional parameters, such as MAGE or preprandial glucose, is also conceivable, as these independent parameters likewise represent risk factors for developing microvascular and macrovascular complications. In this case, the MAGE value may replace the standard deviation of the mean glucose concentration value. The addition of further parameters, such as patient age, and years with diabetes, etc., is also conceivable. The basic model for calculating the area encompassed by the parameters and for normalizing this area against the pentagon for individuals with normal metabolism would remain the same. The Glucose Pentagon 100 may then become a "Glucose Polygon".

While the description above refers to particular embodiments of the present invention, it will be understood that many modifications may be made.

The presently disclosed embodiments are therefore to be considered in all respects as illustrative and not restrictive, the scope of the invention being indicated by the appended claims, rather than the foregoing description.

**Claims**

1. A method of evaluating glycemic control of a patient implemented on a computing device, an infusion device, an infusion device controller / programmer, or a medical device, comprising:

providing a pentagon (100) having five axes radiating from a center of the pentagon, wherein

a first axis (110) has a length representing a range of hemoglobin $A_{1c}$ values,
a second axis (120) has a length representing a range of standard deviation of glucose values,
a third axis (130) has a length representing a range of amount of time per day values exceeding a first limit;
a fourth axis (140) has a length representing a range of daily area- under-curve values exceeding a second limit, and
a fifth axis (150) has a length representing a range of mean glucose values;

plotting a first point (615) on the first axis indicative of a representative hemoglobin $A_{1c}$ value;

plotting a second point (620) on the second axis indicative of a representative standard deviation of glucose value;

plotting a third point (625) on the third axis indicative of a representative amount of time per day value exceeding the first limit;

plotting a fourth point (630) on the fourth axis indicative of a representative daily area-under-curve value exceeding a second limit;

plotting a fifth point (635) on the fifth axis indicative of a representative mean glucose value;

plotting a sixth point (640) on the first axis indicative of a hemoglobin $A_{1c}$ value of the patient;

plotting a seventh point (645) on the second axis indicative of a standard deviation of glucose value of the patient;

plotting an eighth point (650) on the third axis indicative of an amount of time per day value exceeding the first limit of the patient;

plotting a ninth point (655) on the fourth axis indicative of a daily area-under- curve value exceeding the second limit of the patient;

plotting a tenth point (660) on the fifth axis indicative of a mean glucose value of the patient;

determining (665) a first pentagon (101) area formed by the first point (615), the second point (620), the third point (625), the fourth point (630), and the fifth point (635);

determining (670) a second pentagon (201) area formed by the sixth point (640), the seventh point (645), the eighth point (650), the ninth point (655), and the tenth point (660); and

determining (675) a glycemic control parameter (675) based on the first pentagon area (101) and the second pentagon area (201) by dividing the second pentagon area (201) by the first pentagon area (101);

wherein the first axis (110) representing the range of hemoglobin $A_{1c}$ values and the fifth axis (150) representing the range of mean glucose values are adjacent to each other in the pentagon (100).

2. The method of claim 1, wherein the representative hemoglobin $A_{1c}$ value, the representative standard deviation of glucose value, the representative amount of time per day value exceeding the first limit, the representative daily area- under-curve value exceeding the second limit, and the representative mean glucose value are representative of a non-diabetic individual.

3. The method of claim 1, wherein the first limit is 160 mg / dL.

4. The method of claim 1, wherein the second limit is 160 mg/dL.

**Patentansprüche**

1. Verfahren zur Bewertung der glykämischen Kontrolle eines Patienten, das auf einer Rechenvorrichtung, einer Infusionsvorrichtung, einer Infusionsvorrichtungssteuerung / einem Infusionsvorrichtungsprogrammierer oder einer medizinischen Vorrichtung implementiert ist, umfassend:

Bereitstellen eines Fünfecks (100) mit fünf Achsen, die radial von einem Mittelpunkt des Fünfecks ausgehen, wobei

eine erste Achse (110) eine Länge aufweist, die einen Bereich von Hämoglobin $A_{1C}$-Werten darstellt,

eine zweite Achse (120) eine Länge aufweist, die einen Standardabweichungsbereich von Glukosewerten darstellt,

eine dritte Achse (130) eine Länge aufweist, die einen Bereich von Zeitbetrag-pro-Tag-Werten, die eine erste Grenze überschreiten, darstellt;

eine vierte Achse (140) eine Länge aufweist, die einen Bereich von Tageswerten für die Fläche unter der Kurve darstellt, die eine zweite Grenze überschreiten, und

eine fünfte Achse (150) eine Länge aufweist, die einen Bereich von durchschnittlichen Glukosewerten darstellt;

Auftragen eines ersten Punktes (615) auf der ersten Achse, der ein Indikator für einen repräsentativen Hämoglobin $A_{1C}$-Wert ist;

Auftragen eines zweiten Punktes (620) auf der zweiten Achse, der ein Indikator für eine repräsentative Glukosewert-Standardabweichung ist;

Auftragen eines dritten Punktes (625) auf der dritten Achse, der ein Indikator für einen repräsentativen Zeitbetrag-pro-Tag-Wert, der eine erste Grenze überschreitet, ist;

Auftragen eines vierten Punktes (630) auf der vierten Achse, der ein Indikator für einen repräsentativen Tageswert für die Fläche unter der Kurve, der eine zweite Grenze überschreitet, ist;

Auftragen eines fünften Punktes (635) auf der fünften Achse, der ein Indikator für einen repräsentativen durchschnittlichen Glukosewert ist;

Auftragen eines sechsten Punktes (640) auf der ersten Achse, der ein Indikator für einen Hämoglobin $A_{1C}$-Wert des Patienten ist;

Auftragen eines siebten Punktes (645) auf der zweiten Achse, der ein Indikator für eine Standardabweichung des Glukosewerts des Patienten ist;

Auftragen eines achten Punktes (650) auf der dritten Achse, der ein Indikator für einen Wert für den Zeitbetrag pro Tag ist, der die erste Grenze des Patienten überschreitet;

Auftragen eines neunten Punktes (655) auf der vierten Achse, der ein Indikator für einen Tageswert einer Fläche unter der Kurve ist, der die zweite Grenze des Patienten überschreitet;

Auftragen eines zehnten Punktes (660) auf der fünften Achse, der ein Indikator für einen durchschnittlichen Glukosewert des Patienten ist;

Bestimmen (665) einer ersten Fünfeck (101)-Fläche, die durch den ersten Punkt (615), den zweiten Punkt (620), den dritten Punkt (625), den vierten Punkt (630), und den fünften Punkt (635) gebildet wird;

Bestimmen (670) einer zweiten Fünfeck (201)-Fläche, die durch den sechsten Punkt (640), den siebten Punkt (645), den achten Punkt (650), den neunten Punkt (655) und den zehnten Punkt (660) gebildet wird; und

Bestimmen (675) eines glykämischen Kontrollparameters (675) auf Basis der ersten Fünfeckfläche (101) und der zweiten Fünfeckfläche (201) durch Dividieren der zweiten Fünfeckfläche (201) durch die erste Fünfeckfläche (101);

wobei die erste Achse (110), die den Bereich von Hämoglobin $A_{1C}$-Werten darstellt, und die fünfte Achse (150), die den Bereich von durchschnittlichen Glukosewerten darstellt, in dem Fünfeck (100) nebeneinander liegen.

2. Verfahren nach Anspruch 1, wobei der repräsentative Hämoglobin $A_{1C}$-Wert, die repräsentative Standardabweichung des Glukosewerts, der repräsentative Zeitbetrag-pro-Tag-Wert, der die ersten Grenze übersteigt, der repräsentative Tageswert der Fläche unter der Kurve, der die zweite Grenze übersteigt, und der repräsentative durchschnittliche Glukosewert repräsentativ für ein nicht diabetisches Individuum sind.

3. Verfahren nach Anspruch 1, wobei die erste Grenze 160 mg/ dL beträgt.

4. Verfahren nach Anspruch 1, wobei die zweite Grenze 160 mg/dL beträgt.


**Revendications**

1. Procédé d'évaluation de la régulation glycémique d'un patient mis en oeuvre sur un dispositif informatique, un dispositif de perfusion, un dispositif de commande/de programmation de dispositif de perfusion, ou un dispositif médical, comprenant :

la fourniture d'un pentagone (100) ayant cinq axes rayonnant à partir d'un centre du pentagone, dans lequel un premier axe (110) a une longueur représentant une plage de valeurs d'hémoglobine $A_{1C}$, un deuxième axe (120) a une longueur représentant une plage d'écart type de valeurs de glycémie, un troisième axe (130) a une longueur représentant une plage de valeurs de durée par jour dépassant une première limite ;

un quatrième axe (140) a une longueur représentant une plage de valeurs d'aire sous la courbe quotidienne dépassant une deuxième limite, et

un cinquième axe (150) a une longueur représentant une plage de valeurs moyennes de glycémie ;

le traçage d'un premier point (615) sur le premier axe, caractéristique d'une valeur représentative d'hémoglobine $A_{1C}$ ;

le traçage d'un deuxième point (620) sur le deuxième axe, caractéristique d'un écart type représentatif de la valeur de glycémie ;

le traçage d'un troisième point (625) sur le troisième axe, caractéristique d'une valeur de durée représentative par jour dépassant la première limite ;

le traçage d'un quatrième point (630) sur le quatrième axe, caractéristique d'une valeur d'aire sous la courbe quotidienne représentative dépassant une deuxième limite ;

le traçage d'un cinquième point (635) sur le cinquième axe, caractéristique d'une valeur moyenne de glycémie représentative ;

le traçage d'un sixième point (640) sur le premier axe, caractéristique d'une valeur d'hémoglobine $A_{1C}$ du patient ;

le traçage d'un septième point (645) sur le deuxième axe, caractéristique d'un écart type de la valeur de glycémie

12

du patient ;

le traçage d'un huitième point (650) sur le troisième axe, caractéristique d'une valeur de durée par jour dépassant la première limite du patient ;

le traçage d'un neuvième point (655) sur le quatrième axe, caractéristique d'une valeur d'aire sous la courbe quotidienne dépassant la deuxième limite du patient ;

le traçage d'un dixième point (660) sur le cinquième axe, caractéristique d'une valeur moyenne de glycémie du patient ;

la détermination (665) de l'aire d'un premier pentagone (101) formé par le premier point (615), le deuxième point (620), le troisième point (625), le quatrième point (630) et le cinquième point (635) ;

la détermination (670) de l'aire d'un deuxième pentagone (201) formé par le sixième point (640), le septième point (645), le huitième point (650), le neuvième point (655) et le dixième point (660) ; et

la détermination (675) d'un paramètre de régulation glycémique (675) sur la base de l'aire du premier pentagone (101) et de l'aire du deuxième pentagone (201) en divisant l'aire du deuxième pentagone (201) par l'aire du premier pentagone (101) ;

dans lequel le premier axe (110) représentant la plage de valeurs d'hémoglobine $A_{1c}$ et le cinquième axe (150) représentant la plage de valeurs moyennes de glycémie sont l'un à côté de l'autre dans le pentagone (100).

2. Procédé selon la revendication 1, dans lequel la valeur représentative d'hémoglobine $A_{1C}$, l'écart type représentatif de la valeur de glycémie, la valeur représentative de durée par jour dépassant la première limite, la valeur représentative d'aire sous la courbe quotidienne dépassant la deuxième limite et la valeur moyenne de glycémie représentative sont représentatives d'un sujet non diabétique.

3. Procédé selon la revendication 1, dans lequel la première limite est de 160 mg/dL.

4. Procédé selon la revendication 1, dans lequel la deuxième limite est de 160 mg/dL.

FIG. 1

FIG. 2

300

GRP

Risk of developing
diabetic complications

} very high risk

8.0 —

} high risk

7.0 —

6.0 —

} moderate risk

5.0 —

4.0 —

} slightly elevated risk

3.0 —

} low risk

2.0 —

1.30                 no risk

1.0 —                individuals with healthy metabolism

FIG. 3

**FIG. 4A**

FIG. 4B

FIG. 4C

FIG. 5A

FIG. 5B

FIG. 5C

FIG. 5D

PROVIDE GLUCOSE PENTAGON
WITH FIVE AXES.
610

↓

PLOT FIRST POINT ON FIRST AXIS
(HBA1C).
615

↓

PLOT SECOND POINT ON SECOND
AXIS (SD GLUCOSE).
620

↓

PLOT THIRD POINT ON THIRD AXIS
(TIME-DAY > LIMIT).
625

↓

PLOT FOURTH POINT ON FOURTH
AXIS (AUC > LIMIT).
630

↓

PLOT FIFTH POINT ON FIFTH AXIS
(MEAN GLUCOSE).
635

↓

PLOT SIXTH POINT ON FIRST AXIS
(PATIENT HBA1C).
640

PLOT SEVENTH POINT ON
SECOND AXIS
(PATIENT SD GLUCOSE).
645

↓

PLOT EIGHTH POINT ON THIRD
AXIS
(PATIENT TIME-DAY > LIMIT).
650

↓

PLOT NINTH POINT ON FOURTH
AXIS
(PATIENT AUC > LIMIT).
655

↓

PLOT TENTH POINT ON FIFTH AXIS
(PATIENT MEAN GLUCOSE).
660

↓

DETERMINE FIRST PENTAGON
AREA FORMED BY FIRST TO FIFTH
POINTS.
665

↓

DETERMINE SECOND PENTAGON
AREA FORMED BY SIXTH TO
TENTH POINTS.
670

↓

DETERMINE GLYCEMIC CONTROL
PARAMETER BASED ON FIRST
AND SECOND PENTAGON AREAS.
675

FIG. 6

**EP 2 339 953 B1**

**REFERENCES CITED IN THE DESCRIPTION**

**Patent documents cited in the description**

- JP 2004313265 A
- JP 2004164343 A

- JP 2006043360 B